# EUROPEAN PATENT APPLICATION

(11) **EP 4 226 845 A1**
(43) Date of publication of application: **16.08.2023**
(21) Application number: 23155130.0
(22) Date of filing: 06.02.2023
(51) Int. Cl.: A61B 3/15, A61B 3/113, A61B 3/00, A61F 9/00, A61F 9/007

(54) **EYE IMAGE QUALITY ANALYSIS**

(30) Priority: 11.02.2022 CH 1282022
(71) Applicant: Ziemer Ophthalmic Systems AG, 2562 Port (CH)
(72) Inventor: Mönkeberg, Fabian, 2503 Biel (CH); Steinlechner, Michael, 8006 Zürich (CH)
(74) Representative: Rentsch Partner AG

(57) **Abstract**

An ophthalmological image processing device (1) and method are disclosed in which a reference image of an eye of a person is received and analyzed in a processor (11) by calculating a quality measure, the quality measuring being indicative of a suitability of the reference image for a cyclorotation assessment; and an evaluation is performed to determine whether the reference image is suitable for a cyclorotation assessment.

## Description

### FIELD OF THE DISCLOSURE

The present invention relates to an ophthalmological image processing device.

### BACKGROUND OF THE DISCLOSURE

Treatment of a human eye, for example using laser ablation, depends critically on a correct alignment between the eye and the laser such that the correct areas of the eye are treated. The basis of the treatment is defined by a treatment model, which is used to control the laser during treatment. The treatment model is established based on measurements of the eye taken using a diagnostic device.

While setting up the laser for treatment, and also during treatment, it is important to detect an actual position and an actual direction of gaze of the eye such that the laser performs ablation according to the treatment model. In addition, a rotation of the eye, including, for example, a torsion of the eye about an axis (known as cyclorotation) is important to account for, in particular for astigmatic eyes.

Some eyes experience varying degrees of cyclorotation when the axis moves from a horizontal orientation, as is typical when the person is upright, to an inclined position, for example when the person is lying down.

Further, a rotation of the head can also lead to a rotation of the eye.

This presents a challenge during eye treatment, as treatment of the eye, for example using laser ablation, must account for the cyclorotation for best results, in particular for eyes with astigmatism. This is because the treatment model is established based on measurements of the eye taken using a diagnostic device with the person in an upright position, whereas the treatment is usually performed with the person in a supine position. Particularly for people with astigmatism, a treatment model may account for the astigmatism and therefore not be rotationally symmetric.

Known methods for measuring and accounting for a rotation of the eye, in particular a cyclorotation, include manually marking the eyeball of the person when the person is in the upright position and realigning the treatment model according to the mark when the person lies down, the mark having rotated due to the cyclorotation.

Other known methods for accounting for a rotation of the eye rely on a reference image recorded by a diagnostic device when the person is in the upright position, and comparing the reference image with a current image recorded just prior to treatment when the person is in a supine position. This method relies on the reference image being suitable for comparison, which is not always the case.

US7331667B2 describes methods for aligning diagnostic and therapeutic iris images, via iris pattern recognition, for effecting more accurate laser treatment of the eye, in particular using sequential plurality of diagnostic iris images of varying pupil size such that an iris landmark can be tracked between two images. Known methods that automatically align a therapeutic image, taken immediately prior to therapy, to a pre-recorded diagnostic image can pose serious problems in cases where the alignment is, for one reason or the other, not possible. In these cases, the surgeon has no choice but to abort the treatment procedure, move the patient back to an upright position, and mark the eyeball manually as described above. This additional patient handing disrupts the clinical workflow, induces stress to both patient and doctors and increases the risk of the ophthalmic treatment.

### SUMMARY OF THE DISCLOSURE

It is an object of embodiments disclosed herein to provide an ophthalmological image processing device.

In particular, it is an object of embodiments disclosed herein to provide an ophthalmological image processing device comprising a processor configured to receive a reference image of an eye of a person. The processor is configured to analyze the reference image by calculating a quality measure of the reference image, the quality measure indicative of a suitability of the reference image for a cyclorotation assessment. The quality measure comprises an iris visibility measure indicative of a level of visibility of the iris of the eye and/or an iris structure measure indicative of a level of structuring of the iris. The processor is configured to evaluate, using the quality measure, whether the reference image is suitable for a cyclorotation assessment. The processor is configured to generate a message indicating whether the reference image is suitable for a cyclorotation assessment.

In an embodiment, the message includes the quality measure, the iris visibility measure and/or the iris structure measure. As is explained herein, depending on the embodiment, the message further comprises instructions, for example instructions for an eye care professional.

By evaluating and indicating whether the reference image is suitable for a cyclorotation assessment, the ophthalmological image processing device ensures that a subsequent cyclorotation assessment will be successful. The subsequent cyclorotation assessment is performed using the reference image taken immediately prior to the begin of laser treatment, with the patient reclined in a supine position. By ensuring the reference image is of sufficient quality, it can be avoided that the person is required to leave the reclined position and have the eye care professional manually mark his eye (while in an upright position), subsequently return to the reclined position, and have the eye care professional perform the cyclorotation assessment based on the manual mark.

The iris visibility measure is indicative of a level of visibility of the iris, which is an extrinsic property of the iris and depends on the pupil dilation, the degree to which the iris is uncovered by the eyelid and all factors dependent on the photographic characteristics of the reference image (e.g. sharpness and dynamic range). Specifically, the iris visibility measure comprises a photographic quality measure, a pupil dilation measure and/or an eyelid coverage measure. The level of visibility of the iris is therefore subject to changeable conditions regarding how the reference image was taken. Two reference images of the same eye taken under non-identical conditions may therefore have a different level of visibility of the iris.

The iris structure measure is indicative of level of structuring of the iris and is an intrinsic property of the iris given by textures, patterns, lines, features, and/or color variations, of the iris itself. The aforementioned are due to visible anatomical features of the eye, which are present or more prominent in some eyes than others. The level of structuring of the iris is therefore not subject to the conditions regarding how the reference image was taken, and as long as the iris is adequately visible, two reference images of the same eye will have a similar or identical level of structuring.

In an embodiment, the ophthalmological image processing device further comprises a display, wherein the processor is configured to render the message on the display. Optionally, the display is configured to display the reference image alongside the message.

In an embodiment, the processor is further configured to render a warning on the display if the message indicates that the reference image is unsuitable for a cyclorotation assessment. For example, the warning is rendered if the quality measure is below a pre-defined quality measure threshold. Further, in an example, the processor is further configured to display or record instructions for the doctor to manually mark the eye of the patient prior to laser treatment for determining the cyclorotation using the mark, if the reference image is not suitable.

In an embodiment, the reference image is a reference image recorded with the person in an upright position by a camera of a diagnostic device and the processor is configured to receive the reference image from the diagnostic device. In an embodiment, the ophthalmological image processing device is part of the diagnostic device.

In an embodiment, if the reference image is unsuitable for the cyclorotation assessment, the processor is further configured to determine optimization instructions configured to direct the diagnostic device to record a new reference image, and to transmit the optimization instructions to the diagnostic device. The optimization instructions are preferably determined by assessing whether the quality measure is below a pre-defined quality measure threshold.

In a variant, the processor is further configured to receive the new reference image and calculate the quality measure for the new reference image. Preferably, if the iris visibility measure of the quality measure is below a pre-defined iris visibility measure threshold, optimization instructions are determined such that the new reference image has a higher iris visibility measure. The optimization instructions comprise, for example, camera settings, including an exposure time, an aperture, an ISO-setting (sensor gain), a flash illumination setting, and/or a focal depth. Additionally, the optimization instructions can include an instruction to the patient, for example instructing the patient to open his or her eye further, to focus on a particular point, and/or not to blink.

In an embodiment, the processor is further configured to provide the message and the reference image (for example to transmit the message and the reference image, or enable access to the message and the reference image) to an ophthalmological laser treatment device for use in the cyclorotation assessment, in which cyclorotation assessment an angle of cyclorotation of the eye is determined using the reference image and a current image of the eye recorded by a camera of the ophthalmological laser treatment device when the person is in a supine position. For example, the cyclorotation assessment is carried out according to the disclosure of the Swiss patent application No. 70746/2021, which is hereby included in the present disclosure by reference in its entirety.

In an embodiment, the processor is configured to generate the iris visibility measure by analyzing the following photographic characteristics of the reference image: a global dynamic range of the entire reference image, a local dynamic range of one or more areas of the reference image, a global contrast of the entire reference image, a local contrast of one or more areas of the reference image, a global sharpness, a local sharpness of one or more areas of the reference image, a noise level, a reflection indicator indicating whether a reflection of a light source is present, and/or an artifact measure indicating whether visual artifacts are present. The local areas include, for example, the iris, the sclera, the pupil, and/or parts thereof.

In an embodiment, the processor is configured to determine the iris visibility measure by determining a level of pupil dilation of the eye and/or an eyelid coverage of the iris. Preferably, determining the eyelid coverage comprises detecting whether an eyelid of the person is covering the iris of the eye at least partially.

In an embodiment, the processor is configured to generate the iris structure measure by determining whether the iris has global and/or local features which are not rotationally invariant.

In an embodiment, the processor is configured to generate the iris structure measure by identifying one or more landmark features in the reference image, in particular in the iris. In an embodiment, an angular position of the landmark features is identified relative to a center of the eye and a reference line passing through the center of the eye.

In an embodiment, the processor is configured to generate the iris visibility measure and/or the iris structure measure using a neural network.

In an embodiment, the neural network is trained using supervised learning and a training dataset comprising a plurality of training reference images of a plurality of eyes. Each training reference image has an associated label indicating whether the training reference image is suitable for a cyclotorsion assessment. Additionally, or alternatively, each training reference image has an associated pre-determined quality measure, iris visibility measure and/or iris structure measure.

In an embodiment, the neural network is trained using supervised learning and a training dataset comprising a plurality of training reference images, a plurality of corresponding training current images of the eye when the person is in a supine position, and a plurality of corresponding indications of whether a rotation angle of the eye between a given training reference image and a given training current image was determinable (i.e. whether the training reference image is suitable for a cyclotorsion assessment).

In addition to an ophthalmological image processing device, the present disclosure also relates to a method for determining a quality measure of a reference image of an eye comprising a processor of an ophthalmological image processing device receiving a reference image of an eye of a person. The method comprises analyzing the reference image by calculating a quality measure of the reference image, the quality measure indicative of a suitability of the reference image for a cyclorotation assessment. The quality measure comprises an iris visibility measure indicative of a level of visibility of the iris of the eye and/or an iris structure measure indicative of a level of structuring of the iris. The method comprises evaluating, using the quality measure, whether the reference image is suitable for a cyclorotation assessment. The message comprises generating a message indicating whether the reference image is suitable for a cyclorotation assessment.

In addition to an ophthalmological image processing device and a method for determining a quality measure of a reference image, the present disclosure also relates to a computer program product comprising a non-transitory computer-readable medium having stored thereon computer program code for controlling a processor of an ophthalmological image processing device to receive a reference image of an eye of a person. The computer program code controls the processor to analyze the reference image by calculating a quality measure of the reference image, the quality measure indicative of a suitability of the reference image for a cyclorotation assessment. The quality measure comprises an iris visibility measure indicative of a level of visibility of the iris of the eye and/or an iris structure measure indicative of a level of structuring of the iris. The computer program code controls the processor to evaluate, using the quality measure, whether the reference image is suitable for a cyclorotation assessment. The computer program code controls the processor to generate a message indicating whether the reference image is suitable for a cyclorotation assessment.

### BRIEF DESCRIPTION OF THE DRAWINGS

The herein described disclosure will be more fully understood from the detailed description given herein below and the accompanying drawings which should not be considered limiting to the invention described in the appended claims. The drawings in which:
- Fig. 1: shows a block diagram illustrating schematically an ophthalmological image processing device;
- Fig. 2: shows a flow diagram illustrating an exemplary sequence of steps performed by the ophthalmological image processing device;
- Fig. 3a: shows a drawing of diagnostic device configured to record a reference image of an eye of a person in an upright position;
- Fig. 3b: shows a drawing of an ophthalmological laser treatment device configured to record a current image of an eye of a person in a supine position;
- Fig. 4a: shows a drawing of an opened eye;
- Fig. 4b: shows a drawing of a partially covered eye;
- Fig. 5a: shows an image of an eye with an iris having a distinctive structure;
- Fig. 5b: shows an image of an eye with an iris not having a distinctive structure;
- Fig. 6: shows an illustration of an eye of a person;
- Fig. 7: shows a block diagram schematically showing a quality measure;
- Fig. 8: shows a block diagram illustrating an exemplar step for calculating a quality measure;
- Fig. 9: shows a flow diagram illustrating an exemplary sequence of steps for calculating a quality measure; and
- Fig. 10: shows a flow diagram illustrating an exemplary sequence of steps for training a neural network.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

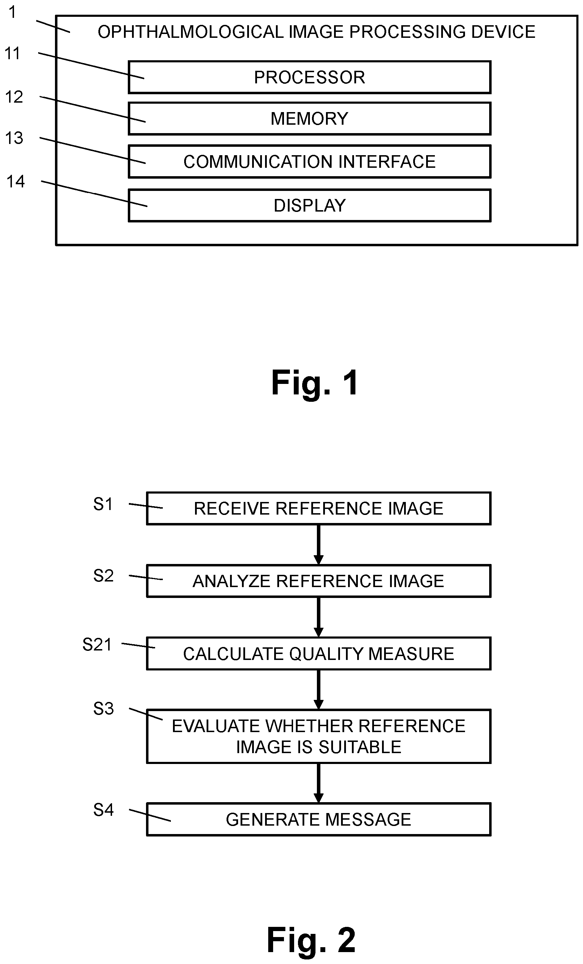

**Figure 1** shows a block diagram illustrating schematically an ophthalmological image processing device 1. The ophthalmological image processing device 1 is a computerized device used by an eye care professional, for example an optometrist, for processing images of eyes of patients. The ophthalmological image processing device 1 comprises one or more processors 11, a memory 12, and a communication interface 13. The processors 11 comprise one or more central processing units (CPUs) and/or other programmable circuits or logic units such as ASICs (Application-Specific Integrated Circuits), for example GPUs (graphics processing units) and TPUs (tensor processing units). The memory 12 comprises volatile and/or non-volatile memory, e.g., random-access memory and/or flash memory having stored thereon program code, data, as well as programmed software modules for controlling the processors 11. Additionally, the memory 12 is configured to store patient data, in particular a reference image 31 of an eye 21 of a person 1. The communication interface 13 is further configured for data communication with one or more external devices. Preferably, the communication interface 13 comprises a network communications interface, for example an Ethernet interface, a WLAN interface, and/or a wireless radio network interface for wireless and/or wired data communication using one or more networks, comprising, for example, a local network such as a LAN (local area network), and/or the Internet.

The skilled person is aware that at least some of the steps and/or functions described herein as being performed on the processor 11 of the ophthalmological image processing device 1 may be performed on one or more auxiliary processing devices connected to the processor 11 of the ophthalmological image processing device 1 using the communication interface 13. The auxiliary processing devices can be co-located with the ophthalmological image processing device 1 or located remotely, for example on an external device, such as a remote server computer (e.g., a cloud-based server).

The skilled person is also aware that at least some of the data associated with the program code (application data) or data associated with a particular person (patient data) and described as being stored in the memory 12 of the ophthalmological image processing device 1 may be stored on one or more auxiliary storage devices connected to the ophthalmological image processing device 1 using the communication interface 13.

The ophthalmological image processing device 1 optionally includes a user interface comprising, for example, one or more user input devices, such as a keyboard, and one or more output devices, such as a display 14. The user interface is configured to receive user inputs from an eye care professional, in particular based on, or in response to, information displayed to the eye treatment professional using the one or more output devices.

Depending on the embodiment, the ophthalmological image processing device 1 is implemented as, or comprises, a personal computer, for example a desktop computer, a laptop computer, a tablet computer, or a smart phone.

In an embodiment, the ophthalmological image processing device 1 is integrated into, or forms part of, an ophthalmological treatment planning device. The ophthalmological treatment planning device is used by the eye care professional for planning an ophthalmological treatment for a patient involving, for example, laser treatment.

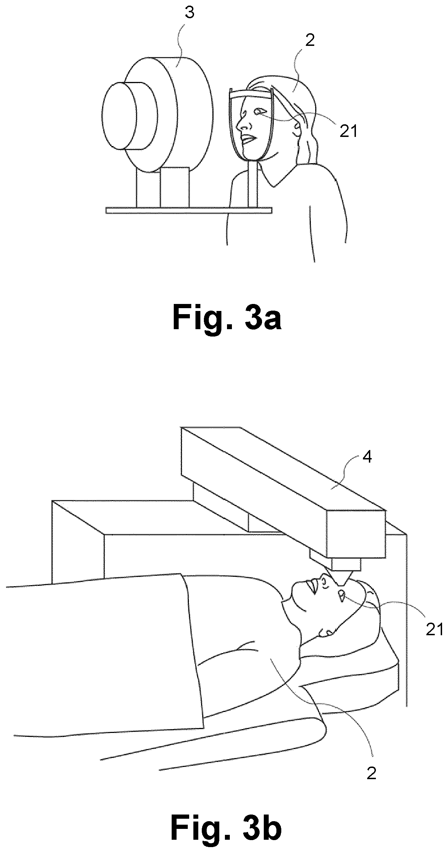

In an embodiment, the ophthalmological image processing device 1 is integrated into, or forms part of, an ophthalmological diagnostic device 3 as is explained in more detail with reference to **Figure 3a**.

In an embodiment, the ophthalmological image processing device 1 is integrated into, or forms part of, an ophthalmological laser treatment device 4 as is explained in more detail with reference to **Figure 3b**.

**Figure 2** shows a flow diagram illustrating an exemplary sequence of steps for determining a quality measure Q of a reference image 31.

In step S1, the ophthalmological image processing device 1 receives the reference image 31. The reference image 31 is a color and/or infrared image of an eye 21 of a person 2. In an embodiment, the reference image 31 comprises interferometric data of the eye 21. Depending on the embodiment, the reference image 31 is received from a component of the ophthalmological image processing device 1, for example the memory 12 (i.e. the reference image 31 is stored in the ophthalmological image processing device 1). Alternatively, the reference image 31 is received via the communication interface 13 from an external device, for example a diagnostic device 3 or a remote server, such as a cloud-based server.

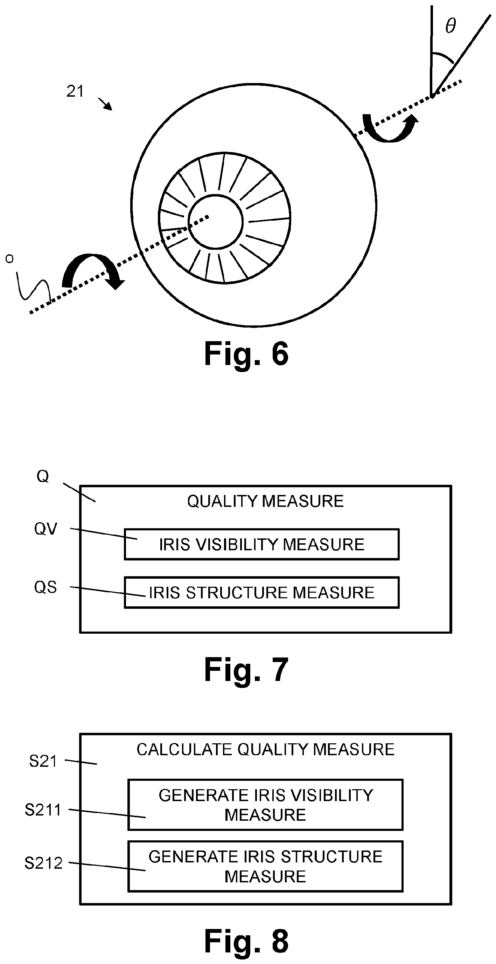

In step S2, the reference image 31 is analyzed. In particular, two types of properties of the reference image 31 are analyzed: extrinsic properties and intrinsic properties. The extrinsic properties relate to properties which are dependent on the conditions under which the reference image 31 was recorded. The intrinsic properties relate to properties inherent to the eye 21 of the person 2. The reference image 31 is analyzed, in the processor 11, by calculating a quality measure Q. The quality measure Q is a quantitative measure, which expresses the suitability of the reference image 31 for use a cyclorotation assessment. The quality measure Q is calculated using an iris visibility measure QV and an iris structure measure QS. For example, the quality measure Q is calculated from the iris visibility measure QV and the structure measure QS using a mathematical function, such as a sum, average, vector norm, and/or geometric mean. The iris visibility measure QV is indicative of a level of visibility of the iris of the eye 21, and the iris structure measure QS is indicative of a level of structuring of the iris, as is explained in more detail with reference to **Figure 7****.**

The quality measure Q, the iris visibility measure QV and/or the iris structure measure QS are, depending on the embodiment, expressed quantitatively, (e.g. using a variable, such as a Boolean variable, a discrete variable, and/or a continuous variable, or a plurality of one or more variables).

In step S3, the processor 11 evaluates whether the reference image 31 is suitable for a cyclotorsion assessment. The suitability is evaluated using the quality measure Q, in particular it is evaluated whether the quality measure Q satisfies a pre-defined threshold. In an example, the components of the quality measure Q (i.e. the iris visibility measure QV and the iris structure measure QS) are individually evaluated to determine whether the reference image 31 is suitable for a cyclotorsion assessment. For example, the processor 11 evaluates whether both the iris visibility measure QV and the iris structure measure QS satisfy a pre-defined iris visibility measure and an iris structure measure threshold, respectively.

In step S4, the processor 11 generates a message indicating whether the reference image 31 is suitable for a cyclotorsion assessment. The message contains, depending on an embodiment, an indication of the quality measure Q and optionally the iris visibility measure QV and/or the iris structure measure QS. The message indicates, depending on the embodiment, qualitative statements indicating whether the reference image 31 is poor, fair, acceptable, or excellent, for example.

In an embodiment, the processor 11 is configured to store the message in the memory 12. The message is stored in association with an identifier of the person 2 and preferably in association with the reference image 31.

In an embodiment, the processor 11 is configured to display the message on the display 14 of the ophthalmological image processing device 1, in particular to display it with prominence such that the eye care professional is immediately made aware of the contents of the message.

In an embodiment, the processor 11 is configured to transmit the message, using the communication interface 13, to an external device, for example a remote server. For example, the processor 11 is configured to transmit the message to a database storing patient records.

**Figure 3a** shows an illustration of a person 2 having a reference image 31 and/or reference interferometric data being recorded by a diagnostic device 3. The person 2, in particular the head of the person 2, is upright, such that the eye 21 of the person is looking in a substantially horizontal direction. The reference image 31 and/or reference interferometric data is recorded prior to treatment of the eye 21. To this end, the diagnostic device 3 comprises a measuring device, for example an imaging measuring device, for example a camera (e.g. comprising a CMOS or CCD chip) configured to record one or more color and/or infrared images, or an interferometric measuring device, for example an OCT (Optical Coherence Tomography) system. The measuring device is configured to record the reference image 31 of the eye 21 and/or record reference interferometric data of the eye 21.

The diagnostic device 3 is configured to record and store the reference image 31 and/or reference interferometric data. The reference image 31 and/or reference interferometric data is then provided to the ophthalmological image processing device 1. For example, the reference image 31 and/or reference interferometric data is transmitted to the ophthalmological image processing device 1 using a data communications network, for example the Internet. Alternatively, the reference image 31 and/or reference interferometric data is stored to a portable data carrier which is then connected to the ophthalmological image processing device 1.

In an embodiment, the ophthalmological image processing device 1 is integrated into, or implemented as, the diagnostic device 3. Preferably, the processor 11 is configured to determine whether the reference image 31 is suitable for the cyclotorsion assessment shortly, more preferably immediately, after the reference image 31 has been recorded. Thereby, for example, the eye care professional operating the diagnostic device 3 is immediately provided with feedback as to whether the reference image 31 is suitable. Should the reference image 31 not be suitable for a cyclotorsion assessment, the eye care professional can retake the reference image 31 while the person 2 is still facing the measuring device. For example, if the person 2 blinked during recording of the reference image 31, the reference image 31 can be retaken to record a new reference image 31.

In an embodiment, the processor 11 of the ophthalmological image processing device 1 is configured to determine optimization instructions, in particular if the evaluation of the reference image 31 indicated a lack of suitability. The optimization instructions are preferably determined using the iris visibility measure QV. The optimization instructions are configured to enable the diagnostic device 3, in particular the measuring device, to record a new reference image 31 with a higher quality measure Q, in particular a higher iris visibility measure QV. The optimization instructions comprise, for example, camera settings and/or flash illumination settings. The camera settings include, for example, an exposure time setting, a lens aperture setting, an ISO-setting (sensor gain setting), and/or a focal depth setting. The flash illumination settings include, for example, a flash power setting.

**Figure 3b** shows an illustration of a person 2 lying in a reclined or a substantially horizontal position for eye treatment. The person 2, in particular the head of the person, is oriented such that the eye 21 looks upwards in a substantially vertical direction. Due to cyclotorsion or other causes, such as a rotation of the head, the eye 21 may be rotated about the rotation angle as is described in more detail with reference to Figure 6. As depicted, the person 2 is lying under an ophthalmological laser treatment device 4. The ophthalmological laser treatment device 4 comprises a laser source, optical elements, a patient interface, and a camera. The camera is arranged such that it can take a current image of the eye 21 of the patient 2 lying in a supine position. The current image of the eye is compared with the reference image 31 during the cyclotorsion assessment. The cyclotorsion assessment uses the reference image 31, recorded of the eye 21 with the person in an upright position, and the current image, recorded of the eye with the person in a supine position, determining an angle of rotation of the eye 21. Thereby, the cyclotorsion assessment determines the degree to which the eye 21 rotates as the person 2 reclines into position under the ophthalmological laser treatment device 4. The angle of rotation of the eye 21 is used to rotate a laser treatment plan such that it is aligned correctly.

In an embodiment, the ophthalmological image processing device 1 is integrated into, or is part of, the ophthalmological laser treatment device 4. The ophthalmological image processing device 1 is in particular configured to generate a message indicating whether the reference image 3 is suitable for a cyclotorsion assessment, prior to the person 2 reclining. In particular, the message indicates to the eye care professional operating the ophthalmological laser treatment device 4 whether a mark is to be applied to the eye 21 of the person 2, such that an angle of rotation is determinable on the basis of a rotation of the mark about a center of the eye 21.

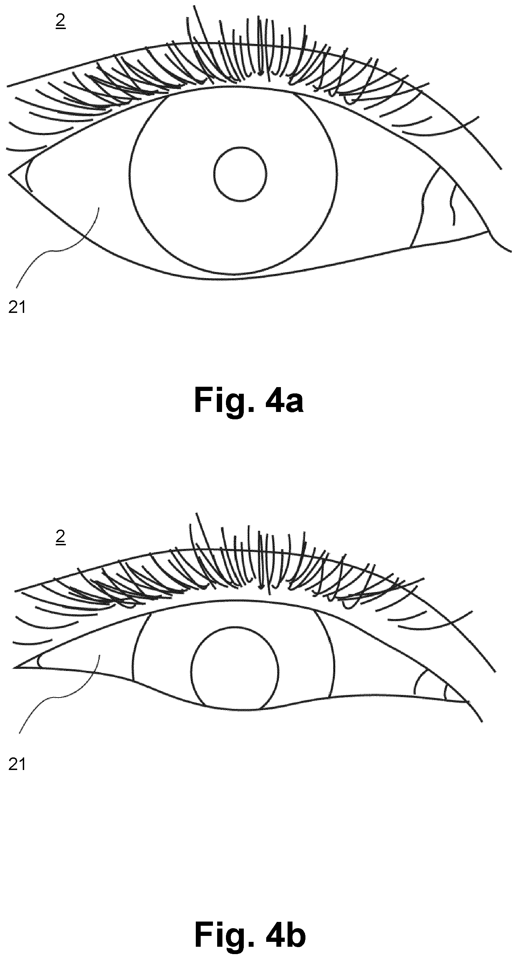

**Figure 4a** shows an illustration of an eye 21 of a person 2 which is opened such that the iris is readily visible. **Figure 4b** shows an illustration of an eye 21 of a person 2 which is partially covered in that the eye lid covers at least part of the iris. The eye 21 shown in **Figure 4a** has an iris visibility measure QV which is relatively higher than the eye 21 shown in **Figure 4b****.**

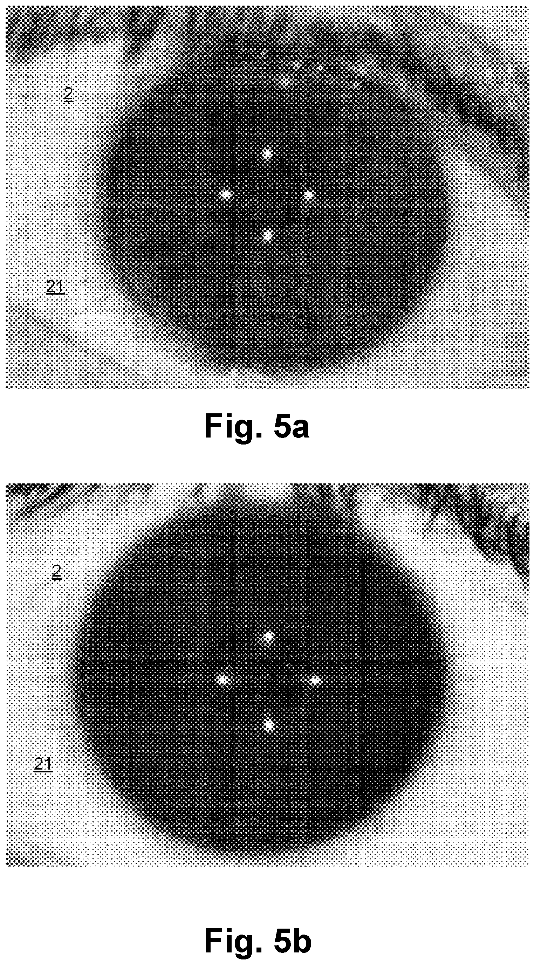

**Figure 5a** shows an illustration of an eye 21 of a person 2 with an iris having a visible structure. Eyes 21 with more visible structure have a higher level of structuring of the iris. The visible structure of an eye 21 arises due to the presence and/or prominence of visible anatomical features. The visible anatomical features include one or more of the following: iris freckles, iris moles, differing pigmentation, particularly in the iris (for example differing pigmentation between a ciliary zone, a pupillary zone, and a peripheral zone of the iris), crypts, and/or radial furrows. Some eyes 21 have more visible structure than other eyes, and eyes 21 with more visible structure are more suitable for a cyclotorsion assessment. **Figure 5b** shows an illustration of an eye 21 of a person with no visible structure.

The eye 21 shown in **Figure 5a** has an iris structure measure QS which is relatively higher than the iris structure measure QS of the eye shown in **Figure 5b**. The iris structure measure QS is substantially independent of the iris visibility measure QV.

In some situations, a poor quality reference image 31 could lead to an eye 21 having a generated iris structure measure QS lower than what it could be with a high quality reference image 31. For example, an out of focus reference image 31 could lead to a lower iris structure measure QS, even if the eye itself has a high level of structuring. A new reference image 31, recorded using different settings, for example an adjusted focus depth, would result in the new reference image 31 having a higher iris structure measure QS. An eye 21 with inherently low structure, however, would not see much, if any, improvement in the iris structure measure QS even under better photographic conditions.

**Figure 6** shows an illustration of an eye 21 having a central axis o about which the eye 21 rotates by the rotation angle θ. In particular, some people 2 experience cyclotorsion which is a rotation of the eye 21 as the head of the person tilts backwards.

**Figure 7** shows a block diagram illustrating schematically the quality measure Q comprising the iris visibility measure QV and the iris structure measure QS. As discussed herein, the iris visibility measure QV is the result of extrinsic properties of the eye 21, such as photographic characteristics and whether or not the eye 21 is partially covered by the eyelid (for example due to squinting or blinking), or not. The iris visibility measure QV comprises a photographic quality measure, which depends on the photographic characteristics, a pupil dilation measure, which depends on a level of pupil dilation, and/or an eyelid coverage measure.

The iris structure measure QS, on the other hand, is a measure of the level of structuring of the iris, i.e. the level of structuring of the intrinsic structure present in the eye 21, in particular the iris. In an embodiment, the level of structuring of the iris includes a level of structuring of the pupil, in particular an edge of the pupil, a center of the pupil, and/or a limbus center. The level of structuring depends on the visible anatomical features of the iris. Eyes 21 having more visible anatomical features, or more visually prominent anatomical features, will typically also have a higher level of structuring. As is also explained with reference to **Figure 5a****,** the visible anatomical features include one or more of the following: iris freckles, iris moles, differing pigmentation, particularly in the iris (for example differing pigmentation between a ciliary zone, a pupillary zone, and a peripheral zone of the iris), crypts, and/or radial furrows.

**Figure 8** shows a block diagram showing further detail of step S21 as described herein. In step S211, the processor 11 generates the iris visibility measure QV. The iris visibility measure QV is generated by analyzing the photographic characteristics. The photographic characteristics of the whole reference image 31, of only a part of the reference image 31 (such as the iris), multiple parts of the reference image (such as the iris and the sclera), or a combination thereof, are analyzed. In an embodiment, the photographic characteristics relate to a dynamic range, with a higher dynamic range leading to an increase of the iris visibility measure QV, a contrast level, with a higher contrast leading to an increase of the iris visibility measure QV, a sharpness level, with a higher sharpness leading to an increase of the iris visibility measure QV, and/or a noise level, with a lower noise level leading to a higher iris visibility measure QV. Additionally, depending on the embodiment, a reflection indicator indicates whether a reflection of a light source (e.g. a flash) is present, and/or a visual artifact.

The individual photographic characteristics are used to generate the iris visibility measure QV. For example, numerical values (preferably normalized values) of the photographic characteristics are combined using a mathematical function to generate the iris visibility measure QV, such as a sum, average, vector norm, and/or geometric mean.

Additionally, in an embodiment, a measure of the coverage of the eye 21 by the eyelid is also used to generate the iris visibility measure QV. In particular, a fully uncovered eye 21 would result in a higher iris visibility measure QV than a partially covered eye 21.

In an embodiment, the processor 11 is configured to determine a level of pupil dilation of the eye 21, and to generate the iris visibility measure QV using the level of pupil dilation. A large pupil necessarily reduces the size of the iris in the reference image 31, and therefore a small pupil size results in a higher iris visibility measure QS.

In step S212, the processor 11 generates the iris structure measure QS. The iris structure measure is generated by identifying, in the reference image 31 of the eye 21, one or more landmark features. The landmark features (also referred to as local features in this disclosure) are localizable, i.e. have a defined location in the iris. The landmark features are due to visible anatomical features of the iris, for example radial furrows, crypts, different colors, or other patterns.

In an embodiment, the processor 11 generates the iris structure measure QS by determining whether there are global features and/or local features in the reference image 31, in particular in the iris, which are not rotationally invariant (i.e. which vary depending on a rotation of the eye. More specifically, some eyes 21 have an iris with regular and repeating patterns which are substantially rotationally invariant, such that the eye 21 looks largely similar if rotated about at least one particular angle. Such an eye 21 may have, at first glance, a visible structure, however is not likely to be suitable for a cyclotorsion assessment as the structures are repeating and self-similar. Therefore, it is advantageous to identify global and/or local features which are, in particular, not rotationally invariant and to generate the iris structure measure QS depending on these features.

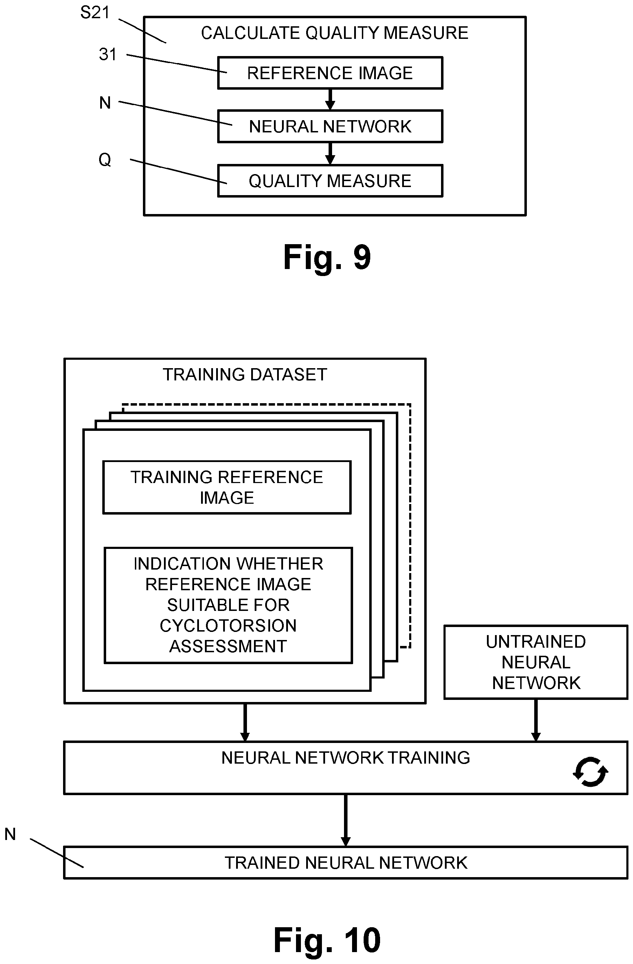

**Figure 9** shows a block diagram of step S21, in which a neural network N is used to generate the quality measure Q. In particular, the processor 11 is configured to provide the reference image 31, or a part thereof, as an input into the neural network N. The neural network N is configured to receive the reference image 31 as an input and provide the quality measure D as an output. The neural network N is implemented in the ophthalmological image processing device 1. In particular, it is stored in the memory 12.

In an embodiment, the neural network N is alternatively implemented in a remote device, for example a cloud-based server, and the ophthalmological image processing device 1 is configured to transmit the reference image 31 to the cloud-based server and receive, from the cloud-based server, the quality measure Q. Depending on the embodiment, the cloud-based server also transmits, to the ophthalmological image processing device 1, the message as described herein.

In an embodiment, the neural network N generates only part of the quality measure Q, in particular the neural network N generates only the iris visibility measure QV or the iris structure measure QS.

In an embodiment, the neural network N generates the iris structure measure QS by identifying local features in the reference image 31, in particular in the iris. The neural network N is configured to generate the iris structure measure QS dependent on a number of local features and/or their distinctiveness.

The neural network N comprises one or more convolutional layers, one or more pooling layers, and one or more activation functions, one or more fully-connected layers, and/or skip connections. Preferably, the neural network N comprises two final and dense fully-connected layers configured to directly output the quality measure Q, the iris visibility measure QV and/or the iris structure measure QS.

Depending on the embodiment, the reference image 31 is pre-processed, before being input to the neural network N, or the neural network N is configured to pre-process the reference image 31. The pre-processing steps comprise image transformations such as a transformation to polar coordinates and/or color adjustments.

In a preferred embodiment, the neural network N is executed by the processor 11 in a GPU and/or in a TPU for faster execution.

The neural network N is a trained neural network N, trained, for example, in the manner shown in **Figure 10**.

**Figure 10** shows a block diagram illustrating an embodiment of how the neural network N is trained.

The neural network N is initialized as an untrained neural network N of a particular architecture with random parameters, e.g. random weights and biases, or with pre-determined parameters, for example configured for image processing tasks. In particular, the training is helped significantly by using readily available pre-trained weights for a InceptionResNetV2 architecture trained on the ImageNet database as a starting point. The training is further expedited by using, for example, the Adam optimizer using a learning rate of 3 · 10⁻⁴.

The untrained neural network is trained using a training dataset comprising a large number, preferably in the order of 1000, of training reference images of a plurality of eyes 21. It is important that the training dataset comprises a wide variety of lighting conditions as well as different eye shapes and iris colors to avoid any bias towards or against different ethnic groups. The training dataset is then used to train the untrained neural network iteratively using supervised learning to generate the trained neural network N. In particular, the training dataset is segregated into a training subset, a test subset, and a validation subset. Data augmentation techniques, for example by producing mirrored and/or rotated copies of training reference images, may be employed to increase the number of training reference images from a more limited initial number of training reference images. The specifics of how the neural network N is trained, and what additional data the training dataset comprises, depends on the embodiment of the invention. Further, the neural network N may be trained by the processor 11 of the ophthalmological image processing device 1 itself, or the neural network N may be trained by an external device and then implemented in the ophthalmological image processing device 1 once trained. Depending on the embodiment, a large amount of computational power is required to train the neural network N, often using specialized neural network software development platforms and associated specialized hardware (e.g. tensor processing units), and it is therefore only feasible to train the neural network N in a cloud-based server having such software and hardware capabilities.

In an embodiment, each training reference image has an associated label indicating whether the training reference image was suitable for a cyclorotation assessment. The neural network N is iteratively trained, using supervised learning, to generate for each training reference image input a quality measure Q output corresponding to whether the training reference image was suitable for a cyclorotation assessment or not.

In an embodiment, each training reference image has an associated pre-determined quality measure Q, iris visibility measure QV, and/or iris structure measure QS, and the neural network N is trained to generate the quality measure Q, iris visibility measure QV, and/or iris structure measure QS, respectively.

In an embodiment, the training dataset comprises a plurality of upright training reference images of a given eye taken when a given person is in an upright position (recorded, for example, using a diagnostic device 3 as described herein), each training reference image having an associated supine training reference image (recorded, for example, using an ophthalmological laser treatment device 4 as described herein) and an indication of whether a rotation angle of the eye depicted was able to be determined (i.e. whether the training reference image was suitable for a cyclorotation or not).

The above-described embodiments of the disclosure are exemplary and the person skilled in the art knows that at least some of the components and/or steps described in the embodiments above may be rearranged, omitted, or introduced into other embodiments without deviating from the scope of the present disclosure.

## Claims

1. An ophthalmological image processing device (1) comprising a processor (11) configured to:
receive (S1) a reference image of an eye (21) of a person (2);
analyze (S2) the reference image (31) by calculating (S21) a quality measure (Q) of the reference image (31), the quality measure (Q) indicative of a suitability of the reference image (31) for a cyclorotation assessment, wherein the quality measure (Q) comprises one or more of: an iris visibility measure (QV) indicative of a level of visibility of the iris of the eye (21), or an iris structure measure (QS) indicative of a level of structuring of the iris;
evaluate (S3), using the quality measure (Q), whether the reference image (31) is suitable for a cyclorotation assessment; and
generate (S4) a message indicating whether the reference image (31) is suitable for a cyclorotation assessment.

2. The ophthalmological image processing device (1) of claim 1, further comprising a display, wherein the processor (11) is configured to render the message on the display (14).

3. The ophthalmological image processing device (1) of claim 2, wherein the processor (11) is further configured to render a warning on the display (14) if the message indicates that the reference image (31) is unsuitable for the cyclorotation assessment.

4. The ophthalmological image processing device (1) of one of claims 1 to 3, wherein the reference image (31) was recorded with the person (2) in an upright position by a camera of a diagnostic device (3) and the processor (11) is configured to receive (S1) the reference image (31) from the diagnostic device (3).

5. The ophthalmological image processing device (1) of claim 4, wherein, if the reference image (31) is unsuitable for the cyclorotation assessment, the processor (11) is further configured to determine optimization instructions configured to direct the diagnostic device (3) to record a new reference image (31), and to transmit the optimization instructions to the diagnostic device (3).

6. The ophthalmological image processing device (1) of one of claims 1 to 5, wherein the processor (11) is further configured to provide the message and the reference image (31) to an ophthalmological laser treatment device (4) for use in the cyclorotation assessment, in which cyclorotation assessment an angle of cyclorotation of the eye (21) is determined using the reference image (31) and a current image of the eye (21) recorded by a camera of the ophthalmological laser treatment device (4) when the person (2) is in a supine position.

7. The ophthalmological image processing device (1) of one of claims 1 to 6, wherein the processor (11) is configured to generate (S211) the iris visibility measure (QV) by analyzing one or more of the following photographic characteristics of the reference image (31): a global dynamic range of the entire reference image (31), a local dynamic range of one or more areas of the reference image (31), a global contrast of the entire reference image (31), a local contrast of one or more areas of the reference image (31), a global sharpness, a local sharpness of one or more areas of the reference image (31), a noise level, a reflection indicator indicating whether a reflection of a light source is present, or an artifact measure indicating whether visual artifacts are present.

8. The ophthalmological image processing device (1) of one of claims 1 to 7, wherein the processor (11) is configured to generate (S211) the iris visibility measure (QV) by determining one or more of: a level of pupil dilation of the eye (21), or an eyelid coverage of the iris.

9. The ophthalmological image processing device (1) of one of claims 1 to 8, wherein the processor (11) is configured to generate (S212) the iris structure measure by determining whether the iris has global and/or local features which are not rotationally invariant.

10. The ophthalmological image processing device (1) of one of claims 1 to 9, wherein the processor (11) is configured to generate (S212) the iris structure measure (QS) by identifying one or more landmark features in the reference image (31) of the eye (21).

11. The ophthalmological image processing device (1) of one of claims 1 to 10, wherein the processor (11) is configured to generate (S211, S212) one or more of: the iris visibility measure (QV), or the iris structure measure (QS), using a neural network (N).

12. The ophthalmological image processing device (1) of one of claims 1 to 11, wherein the neural network is trained using supervised learning and a training dataset comprising a plurality of training reference images of a plurality of eyes (21), each training reference image having an associated pre-determined quality measure (Q), iris visibility measure (QV), and/or iris structure measure (QS).

13. The ophthalmological image processing device (1) of one of claims 1 to 12, wherein the neural network (N) is trained using supervised learning and a training dataset comprising a plurality of training reference images, a plurality of corresponding training current images of the eye (21) when the person (2) is in a supine position, and a plurality of corresponding indications of whether a rotation angle of the eye (21) between a given training reference image and a given training current image was determinable.

14. A method for determining a quality measure (Q) of a reference image (31) of an eye (21) comprising a processor (11) of an ophthalmological image processing device (1) performing the steps of:
receiving (S1) a reference image (31) of an eye (21) of a person (2);
analyzing (S2) the reference image (31) by calculating (S21) a quality measure (Q) of the reference image (31), the quality measure (Q) indicative of a suitability of the reference image (31) for a cyclorotation assessment, wherein the quality measure (Q) comprises one or more of: an iris visibility measure (QV) indicative of a level of visibility of the iris of the eye (21), or an iris structure measure (QS) indicative of a level of structuring of the iris;
evaluating (S3), using the quality measure (Q), whether the reference image (31) is suitable for a cyclorotation assessment; and
generating (S4) a message indicating whether the reference image (31) is suitable for a cyclorotation assessment.

15. A computer program product comprising a non-transitory computer-readable medium having stored thereon computer program code for controlling a processor (11) of an ophthalmological image processing device (1) to:
receive (S1) a reference image (31) of an eye (21) of a person (2);
analyze (S2) the reference image (31) by calculating (S21) a quality measure (Q) of the reference image (31), the quality measure (Q) indicative of a suitability of the reference image (31) for a cyclorotation assessment, wherein the quality measure (Q) comprises one or more of: an iris visibility measure (QV) indicative of a level of visibility of the iris of the eye (21), or an iris structure measure (QS) indicative of a level of structuring of the iris;
evaluate (S3), using the quality measure (Q), whether the reference image (31) is suitable for a cyclorotation assessment; and
generate (S4) a message indicating whether the reference image (31) is suitable for a cyclorotation assessment.
